# EUROPEAN PATENT SPECIFICATION

(11) **EP 0 727 184 B1**
(45) Date of publication and mention of the grant of the patent: **11.12.2002**
(21) Application number: 96101808.2
(22) Date of filing: 08.02.1996
(51) Int. Cl.: A61B 5/042

(54) **A catheter, particularly for the treatment of cardiac arrhythmia**
Katheter, besonders zur Behandlung von Herzarrhythmie
Cathéter, notamment pour le traitement de l'arhythmie cardiaque

(30) Priority: 15.02.1995 IT TO950104
(43) Date of publication of application: 21.08.1996
(73) Proprietor: SORIN BIOMEDICA CARDIO S.p.A., 13040 Saluggia (Vercelli) (IT)
(72) Inventor: Rolando, Giovanni, I-10034 Chivasso (Torino) (IT); Garberoglio, Bruno, I-10156 Torino (IT)
(74) Representative: Bosotti, Luciano

(56) References cited:
- WO-A-94/07411
- WO-A-94/21168
- WO-A-95/08948
- US-A- 5 255 679

## Description

The present invention relates to catheters usable for the treatment of cardiac arrhythmia.

It is known that the heart muscle, the function of which is essentially to act as a pump to maintain the circulation of the blood in the body, may be subject to alterations in the contraction mechanism, generally known as arrhythmia, which may affect the various chambers of the heart. In particular, arrhythmia may affect the left ventricle, that is, the part of the heart the function of which is to force the blood into the circulatory system.

The contractions of the heart muscle upon which the operation of the heart is based result from the application of electrical waves. In deliberately simplified terms, these waves can be seen as being generated in the atrium and being propagated through the atrio-ventricular connection towards the ventricles, then proceeding in a centrifugal direction through the conductive tissue of the endocardium.

Essentially, the abnormality defined as arrhythmia can be attributed to a change in the propagation of the excitation wave through the heart muscle. In deliberately simplified terms (but according to concepts well known in the medical field) arrhythmia can be attributed essentially to local short-circuiting of the propagation of the excitation waves. These cause the muscle to be excited and to contract before this is actually required for the heart function to be performed correctly.

The regions of the heart wall in which arrhythmia phenomena are triggered (which are usually located in the endocardium but may also be situated in the thickness of the myocardium) are currently known as arrhythmogenesis sites or foci (or ectopic foci).

In addition to pharmacological treatment which has intrinsic disadvantages, a treatment for arrhythmia which is now very widespread provides for the location of the foci within the heart chamber concerned by an operation currently known as "mapping" and the subsequent application of localized energy (for example heat or electromagnetic energy) so as to bring about passivation of the arrhythmogenesis site by an operation currently known as "ablation".

In recent years, extensive research activities have been dedicated to solving the problem of rendering both the mapping operation and the ablation operation effective and reliable (particularly as regards speed of operation and the precision of the operation which is decisive in clearing up the arrhythmic syndrome). This particularly concerns the possibility of performing these operations by means of catheterization according to a solution which is now established as greatly preferable in order to avoid the need to perform open chest surgery.

This research activity is documented by a corresponding number of patent documents, amongst which may be cited, by way of example, the documents US-A-4 522 212, US-A-5 237 996, US-A-4 293 869, US-A-5 345 936, EP-A-0 573 311 and WO-A-94/12098.

These prior documents address in detail some specific problems such as the construction of the mapping and/or ablation electrodes, the structure of the respective electrical connections, even along the respective catheters, the criteria which may be adopted for processing the signals, the generation and the transfer of the ablation energy towards the application site, etc.

Problems of various types continue to arise, however, in the production of catheters of the type specified above.

In the first place, an accurate mapping operation, particularly as regards precision and resolution in the identification of the ectopic foci presupposes that the mapping electrodes can be brought as much as possible into contact with the wall of the heart chamber investigated. From this point of view there is a certain conflict between the need to give the element of the catheter which carries the electrodes a certain ''body" so that it can be manoeuvred at its proximal end in order to bring about an effective movement of the electrodes, and the need to ensure that the structures which support the electrodes are extremely flexible so as to be able to follow the shape of the heart chamber wall which is very uneven.

In this connection, it should not be forgotten that the mapping and ablation operations are normally carried out whilst conditions of tachycardia (typical conditions in which arrhythmia occurs) are induced in the patient and hence with the heart muscle subjected to intense excitation.

As far as the ablation electrode is concerned, there is therefore a need to be able to arrange for it to be brought rapidly and precisely to the ectopic site on which the ablation is to be carried out.

Precisely for the reasons explained above (that is, the fact that the operation is carried out whilst tachycardia is induced in the patient) it is essential to ensure that the entire operation (mapping/ablation) can be carried out in a short time, nominally of the order of minutes and not, as frequently still occurs, several hours.

The present invention primarily addresses the problem of providing a catheter structure having mapping means which are improved both as regards the identification of the ectopic foci and, in a preferred embodiment of the invention, as regards the possibility of integrating the ablation function in the catheter so as to create a single system which can effectively perform both the operations considered.

According to the present invention, this object is achieved by virtue of a catheter having the specific characteristics recited in claim 1.

The invention will now be described, purely by way of non-limiting example, with reference to the appended drawings, in which:
Figures 1 to 3 show the general structure of the distal portion of a catheter to which the invention is applied, with reference to two different possible embodiments shown in Figures 2 and in Figure 3, respectively,
Figures 4 and 5 show schematically the carrying-out of the mapping operation with the use of a catheter such as that shown in Figure 2,
Figure 6 shows schematically the ablation operation being carried out on the ectopic foci on the atrio-ventricular septum,
Figure 7 shows, in detail, part of the structure of a catheter according to the invention,
Figure 8 shows schematically the configuration of the ablation portion of a catheter according to the invention,
Figures 9 to 11 are transverse sections of a combined mapping/ablation catheter formed according to the invention, and
Figures 12 and 13 show schematically the positioning of the ablation catheter in a combined mapping/ablation system in which the mapping portion is formed according to the configuration of Figure 2 (Figure 12) and according to Figure 3 (Figure 13), respectively.

As a basic premise, it should be remembered that the present description relates primarily to the support structure of the mapping and ablation electrodes of the catheter. For this reason, the specific constructional details of the individual mapping electrodes, the respective electrical and functional connections, as well as the structure and the connections of the ablation electrode present in some preferred embodiments of the invention are not described in detail herein, since they are not relevant *per se* for an understanding and the implementation of the invention. For a general description of these concepts, reference may usefully be made to the prior documents cited in the introductory portion of the present description.

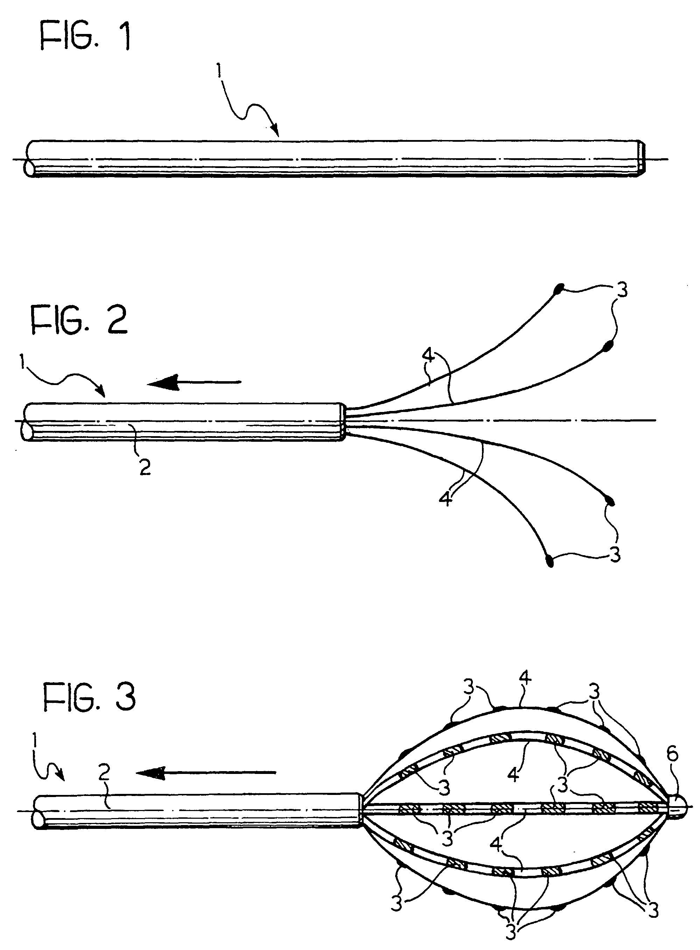

In Figure 1, a catheter according to the invention, generally indicated 1, is shown in the closed position, that is, in the position in which (according to widely known criteria) its distal portion, which is the portion shown in Figure 1, is passed into one of the chambers of a patient's heart in order to perform treatment against arrhythmia. According to known criteria (see the introductory part of the present description) this operation consists of a mapping stage (the location of ectopic foci) and an ablation stage (the elimination of the foci as a result of the localized application of energy).

The catheter 1 can be regarded as being constituted essentially by an outer sheath or shaft 2 from which a plurality of mapping electrodes branch out and can be uncovered as a result of an axial retraction by the operator from the proximal end of the catheter which is kept outside the patient's body.

In particular, in the catheter 1 shown in Figure 2 the solution adopted for the mounting of the mapping electrodes 3, is a cluster- or fountain-like solution such as that described, for example in the documents US-A-4 522 212 or US-A-5 237 996, in which each mapping electrode 3 is mounted on the end of a respective resilient rod 4.

In the catheter shown in Figure 3, on the other hand, the solution adopted is of the type described in the documents US-A-5 293 869 or EP-A-0 573 311, in which the electrodes 3 are arranged in an orderly manner in groups on respective arcuate elements 4 which branch out from the shaft 2 of the catheter and are connected in a connection head 6 at their distal ends so as jointly to define a cage- or basket-like structure.

As will be appreciated better from the following, the solution according to the invention is suitable for application to both of the mapping geometries described above to allow correct positioning of an ablation electrode introduced (at the same time as the mapping electrodes or at a subsequent stage) into the heart chamber in which the treatment is carried out.

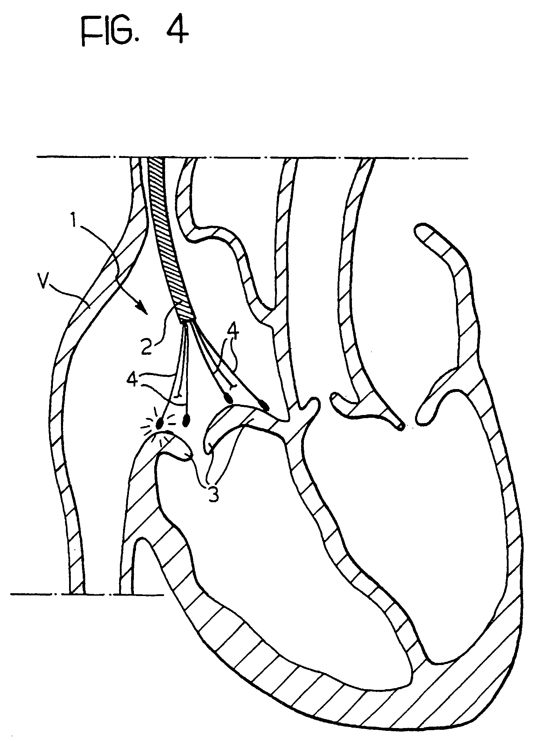

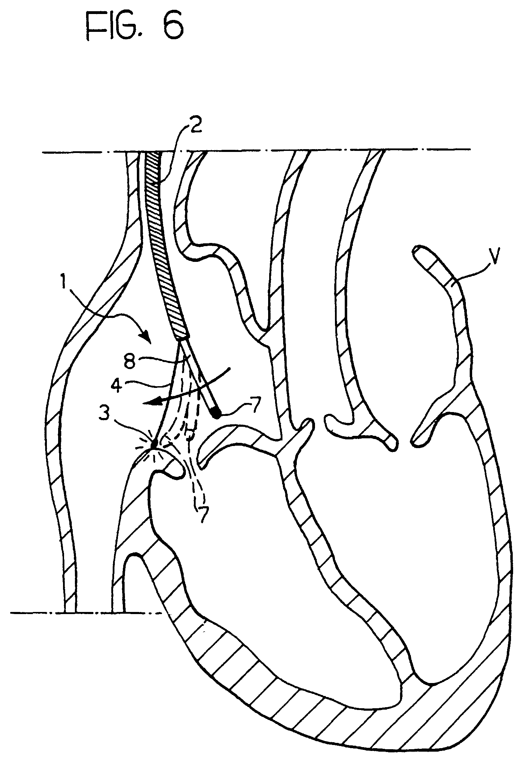

By way of example, Figures 4 to 6 show a typical arrangement of the mapping portion of a catheter of the type illustrated in Figure 2 in a chamber of the heart, generally indicated V.

Specifically, Figure 4 shows the location of the catheter 1 used to locate ectopic foci on the atrial side of the right atrio-ventricular septum, at the annulus of the tricuspid valve.

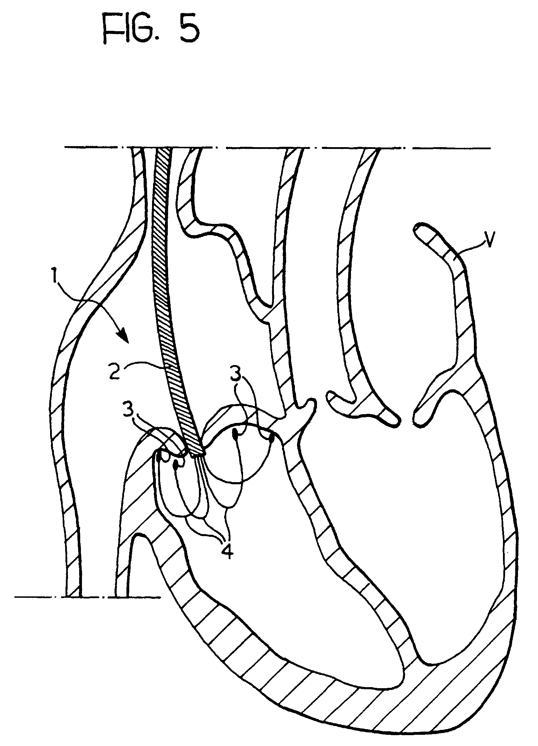

Figure 5 shows the way in which the catheter 1 is located at the same site but on the ventricular side.

It will be appreciated that the correct location of the electrodes 3 achieved by the resilient rods 4 plays an important part in both cases. This applies in particular to the operation on the ventricular side (Figure 5) in which it is necessary, particularly with the use of a catheter structure such as that shown in Figure 2, for the rods 4 to be able to adopt an extremely arcuate attitude, once they have passed the atrio-ventricular septum, in order to be able to bring the electrodes 3 against the annulus of the valve.

This can be achieved by known criteria, particularly as regards the selection of the materials constituting the rods 4 (materials having superelastic properties, etc.).

The invention specifically addresses a problem which arises after the introduction and the location of the mapping electrodes 3, that is, the problem of accurate and quick positioning of the.ablation electrode 7 at the arrhythmogenesis site identified by a corresponding electrode 3.

In general, (again according to a solution known from several of the prior documents already cited several times in the introductory part of the present description), the ablation catheter, indicated 7, is mounted at the distal end of a respective support element or member 8 which can be made to penetrate axially along the catheter 1 (during the introduction of the catheter itself into the heart cavity or at a subsequent stage). The support member 8 can be seen as a type of finger, the distal end of which projects from the distal end of the shaft 2 with the ability to be oriented (under the effect of known movement means) so as to assume a more or less arcuate or bent configuration in order to position the ablation electrode 7 in close proximity to the operation site located by a corresponding electrode 3.

As already stated, it is important that this operation can be carried out with maximum accuracy and speed.

Precision is important since the ablation electrode 7 is required to be able to intervene and perform its own operation at the site which has been located by means of a corresponding electrode 3.

Speed is important since the operation is carried out whilst the patient is kept in conditions which favour the onset of arrhythmic phenomena, typically in tachycardia conditions; it is therefore desirable for this anomalous condition, which is usually induced by the administration of drugs, to be restricted to short periods.

Specifically, the invention addresses the problem of ensuring that, once the electrode 3 corresponding to the site at which the ablation operation is to be carried out has been identified during the mapping stage, the ablation catheter 7 can be oriented accurately and quickly to bring the ablation electrode to a position coinciding with that of the mapping electrode 3 identifying the operation site.

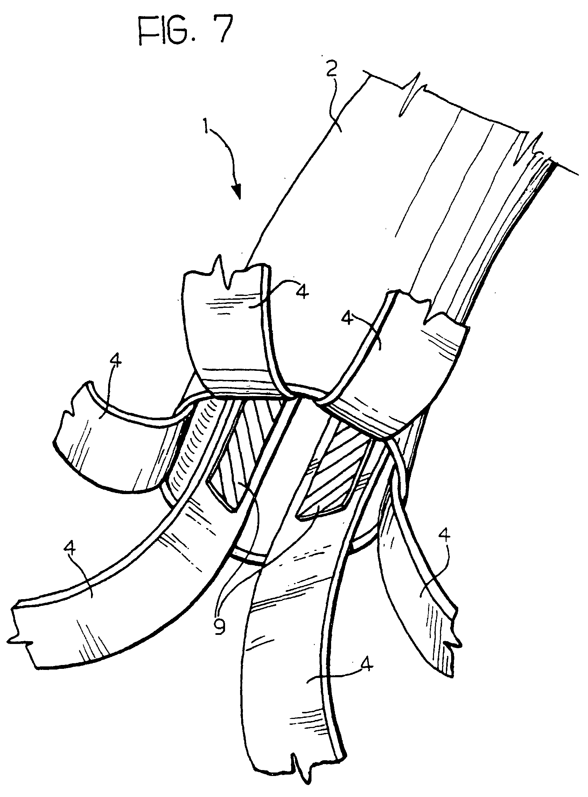

Figure 7 shows in detail the part of a catheter 1 according to the invention which is located at the connection between the shaft (the sheath) 2 and the base portions of the resilient elements 4 which carry the mapping electrodes 3 (not visible in Figure 7) at their distal ends (in the case of the solution of Figure 2) or along their lengths (according to the solution of Figure 3).

According to an embodiment of the invention which up to now has been found preferable, the resilient elements 4 which diverge radially from the shaft are produced in the form of small plates or bars comparable, to a certain extent, to small leaf springs formed (in known manner) of a material having superelastic properties.

The use of plates or bars has the advantage of conferring on each element 4, on the one hand, considerable flexibility in the plane perpendicular to the transverse axis of the plate or bar and, on the other hand, a certain stiffness with regard to any bending or twisting relative to that plane.

According to an important characteristic of the invention, each of the elements 4, which are preferably spaced equiangularly at the outlet opening of the shaft 2 carries, either directly or as a result of the application of an insert, a respective electrical contact 9 connected (by means of conductor elements not shown specifically in the drawing) to the proximal end of the catheter 1 where the operator is situated.

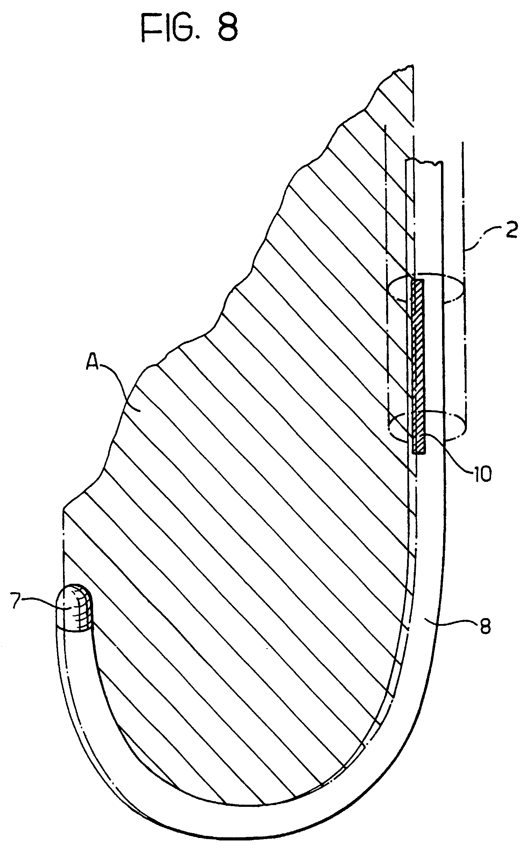

The electrical contacts 9 which are also preferably distributed equiangularly around the periphery of the shaft 2 of the catheter 1 jointly define a cavity along which the support element 8 which carries the ablation electrode 7 at its end (Figure 8) can be introduced as far as the distal end of the catheter.

The element 8 is produced, in known manner, in the form of a type of finger or stalk which, starting from a generally straight rest configuration, can be bent so as to adopt a more or less curved, hook-like position; this is achieved as a result of a bending operation in a plane generally indicated A in Figure 8. This result can be achieved by various possible solutions which are not described in detail herein since they are not relevant *per se* for the purposes of an understanding of the implementation of the invention; in the simplest case, the desired bending is achieved by means of a tie mounted eccentrically relative to the element 8 and manoeuvrable from the proximal end of the catheter.

On its base portion which is intended to be kept at the distal end of the shaft 2 of the catheter, the element 8 carries a respective elongate contact 10 which extends in the plane A, is applied to the element 8 - as in the case of the contacts 9 - by evaporation under vacuum, or metallization techniques, etc., and is situated, relative to the body of the element 8, on the side thereof which corresponds to (or possibly is opposite, in which case the angular datum will be interpreted as being offset by 180°) the side or flank on which the element 8 bends like a hook in the plane A.

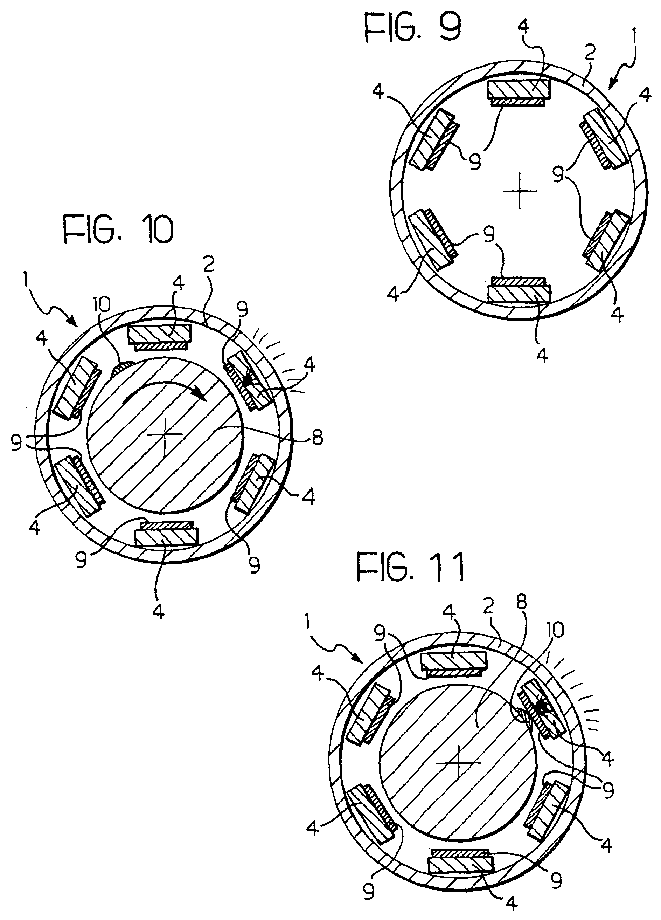

The various sectioned views of Figures 9 to 11 can be considered essentially as sections of the catheter 1 taken in a diametral plane approximately corresponding to the distal opening of the shaft 2; in addition to the shaft or sheath 2, the resilient elements 4 which carry the mapping electrodes 3 as well as the contacts 9 which are on the inner faces thereof, facing towards the axial cavity of the catheter 1, are clearly recognizable. The element 8 which carries the ablation electrode 7 is advanced in this cavity until the contact 10 is brought to the distal opening of the shaft 2, that is, to an axial position facing the contacts 9.

In the embodiment described herein, there are six resilient elements 4 carrying a corresponding number of contacts 9. It should, however, be understood that this selection is in no way essential for the purposes of the implementation of the invention.

In particular, Figure 10 relates to a situation in which the catheter 1 may be arranged upon completion of the mapping stage which is concluded when one of the electrodes 3 is identified, by processing and analysis of the signals supplied simultaneously by the various electrodes 3, as the electrode which is located at a possible ablation site.

This situation is shown schematically in Figure 10, in which the element 4 which in fact carries the mapping electrode (not shown) which is located at the ablation site is marked with an asterisk.

At this point, the operator manoeuvring the catheter 1 from its proximal end can orient the element 8 (which has already been introduced into the sheath 2 beforehand or is introduced at that moment) by rotating it inside the shaft 2 until the contact 10 is brought into physical and electrical contact with the conductive track 9 disposed on the resilient element 4 carrying the electrode 3 which is located at the ablation site. This orientation operation can be performed by the operator quickly (for example, by means of a servo-motor) and safely; when the desired orientation is reached, the contact 10 which is also connected (by means of a conductor not shown specifically in the drawings) to the proximal end of the catheter in fact closes the electrical circuit with the corresponding contact 9 giving rise to an electrical contact (closed circuit) condition which can be identified clearly from the proximal end of the catheter.

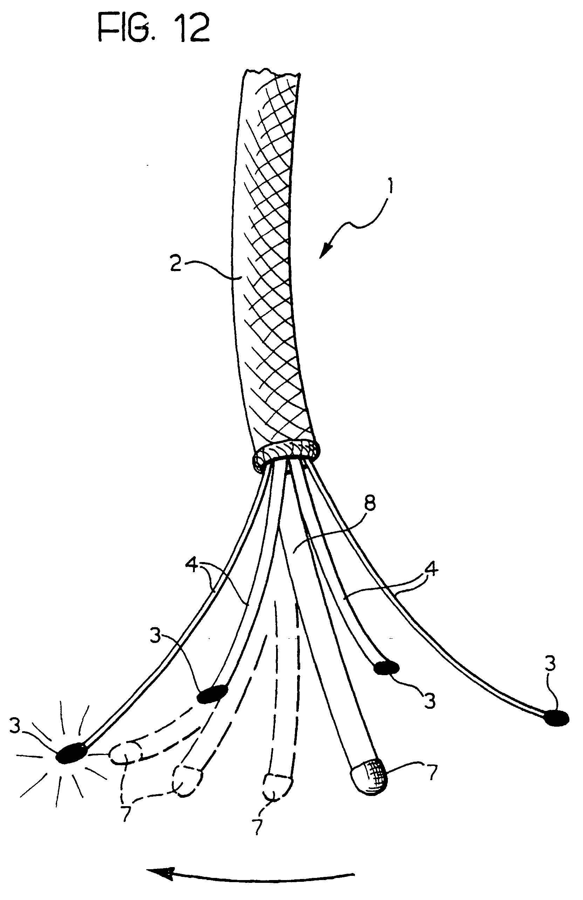

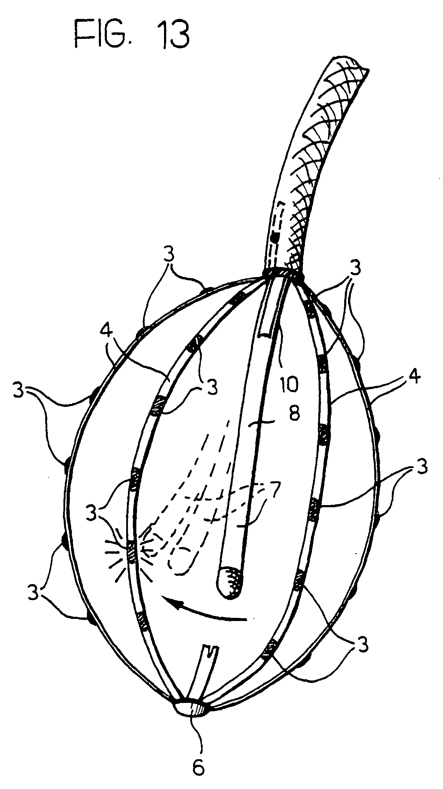

At this point, the element 8 carrying the ablation electrode 7 can be manoeuvred and operated (in known manner) so as to be bent in the general plane A with the certainty that the element 8 is oriented angularly precisely in the direction of the electrode 3 which has been located at the operation site and is mounted on the respective resilient element. 4 - which is a plate or bar with little capability to bend or twist from the general plane in which it lies. This orientation movement is shown schematically by broken lines in Figures 12 and 13. The element 8 can also be adjusted by being slid slightly axially along the sheath 2 of the catheter.

Naturally, the foregoing description (the arrangement of a single contact 10 on the element 8 carrying the ablation catheter 7) relates to only one possible embodiment of the invention.

For example, it would be possible to provide, on the periphery of the element 8, as many electrical contacts 10 as there are contacts 9 and hence resilient elements 4 so as to be able to establish, for each angular position, a corresponding unambiguous correspondence between contacts on the element 8 and contacts on the resilient elements 4. Moreover, as already stated incidentally above, the contact 10 need not be located on the side towards which the element 8 bends but could be on the opposite side; in this case, the angular datum found would be offset by 180° from that desired; the datum of the desired bending plane would in any case be provided, regardless of a fixed angular displacement.

Moreover, although the description given above with reference to the appended drawings relates to contacts 9 and 10 located at the distal opening of the shaft 2, naturally, a different axial position along the axis of the catheter may be selected, for example, so as to move the contacts 9 and 10 towards the proximal end thereof.

Naturally, the principle of the invention remaining the same, the details of construction and forms of embodiment may be varied widely with respect to those described and illustrated, without thereby departing from the scope of the present invention. This applies particularly with regard to the solution used for the mounting of the mapping electrodes 3; in this connection, reference may usefully be made to the various configurations illustrated in the various prior documents cited several times in the course of the present description.

## Claims

1. A catheter for the treatment of cardiac arrhythmia, comprising:
- a catheter shaft (2) the distal end of which can be brought into a heart chamber for operation (V),
- a plurality of mapping electrodes (3) carried by resilient elements (4) branching out from the distal end of the shaft (2) and lying in respective planes, and
- an ablation electrode (7) mounted on a respective support element (8) which can be located in a generally central position relative to the resilient elements (4) and can bend in at least one respective bending plane (A) in order to move the ablation electrode (7) towards an operation site identified by one of the mapping electrodes (3),
- the support element (8) is generally orientable relative to the shaft (2) so that the bending plane (A) can be aligned selectively with the planes containing the resilient elements (4),
**characterized in that**:
- the resilient elements (4) carry respective associated angular location electrical contacts (9), and
- the support element (8) of the ablation electrode (7) carries at least one respective electrical contact (10) which can be brought selectively to positions in which it is in electrical contact with the location electrical contacts (9) of the resilient elements (4) as a result of the orientation of the support element (8), each position in which the at least one further respective electrical contact (10) is in electrical contact with a respective location electrical contact (9) identifying the alignment of the bending plane (A) with one of the planes containing a resilient element (4), the arrangement enabling the bending plane (A) to be aligned selectively, in use, with the plane containing the resilient element (4) which carries the mapping electrode (3) identifying the operation site.

2. A catheter according to Claim 1, **characterized in that** the resilient elements (4) are produced in the form of plate or bar elements which show a certain resistance to bending (twisting) from the respective planes containing them.

3. A catheter according to Claim 1 or Claim 2, **characterized in that** the resilient elements (4) branch out from the shaft (2) of the catheter in a generally cluster- or fountain-like configuration.

4. A catheter according to Claim 1 or Claim 2, **characterized in that** the resilient elements (4) branch out from the shaft (2) of the catheter (1) and then converge at their distal ends at a common connection point (6) in a generally cage- or basket-like configuration.

5. A catheter according to any one of the preceding claims, **characterized in that** the location electrical contacts (9) are associated with the radially inner faces of the resilient elements (4).

6. A catheter according to any one of the preceding claims, **characterized in that** the resilient elements (4) are spaced equiangularly around the catheter (1).

7. A catheter according to any one of the preceding claims, **characterized in that** the respective location electrical contacts (9) and the further electrical contact (10) are situated adjacent the distal opening of the shaft (2) of the catheter.

8. A catheter according to any one of the preceding claims, **characterized in that** the support element (8) of the ablation electrode (7) has a single further respective electrical contact (10) extending in the bending plane (A).

9. A catheter according to Claim 8, **characterized in that** the further respective electrical contact (10) is located on the side of the support element (8) on which the bending movement takes place.

10. A catheter according to any one of the preceding claims, **characterized in that** the length of the support element (8) of the ablation electrode (7) is selectively adjustable.

## Patentansprüche

1. Katheter zur Behandlung kardialer Arrhythmien, mit
einem Katheterschaft (2), dessen distales Ende bei einer Operation (V) in eine Herzkammer einführbar ist,
mehreren Abbildungselektroden (3), die durch elastische Elemente (4), die von dem distalen Ende des Schaftes (2) abzweigen und sich in entsprechenden Ebenen erstrecken, gehalten sind, und
einer Ablationselektrode (7), die an einem entsprechenden Halteelement (8) befestigt ist, das in einer im wesentlichen zentralen Position relativ zu den elastischen Elementen (4) positionierbar und in wenigstens einer entsprechenden Biegeebene (A) biegbar ist, um die Ablationselektrode (7) in Richtung eines durch die Abbildungselektroden (3) identifizierten Operationssitus zu bewegen,
wobei das Halteelement (8) im wesentlichen relativ zum Schaft (2) ausrichtbar ist, so daß die Biegeebene (A) wahlweise in Flucht mit den die elastischen Elemente (4) enthaltenen Ebenen positionierbar ist,
**dadurch gekennzeichnet, daß**
die elastischen Elemente (4) entsprechend zugeordnete, winklig angeordnete elektrische Lokalisierungskontaktelemente (9) aufweisen, und
das Halteelement (8) der Ablationselektrode (7) wenigstens ein entsprechendes elektrisches Kontaktelement (10) hält, das wahlweise in Stellungen bewegbar ist, in denen es infolge der Ausrichtung des Halteelementes (8) im elektrischen Kontakt mit den elektrischen Lokalisierungskontaktelementen (9) der elastischen Elemente (4) steht, wobei anhand jeder Stellung, in der das wenigstens eine weitere elektrische Kontaktelement (10) im elektrischen Kontakt mit einem entsprechenden elektrischen Lokalisierungskontaktelement (9), das die Ausrichtung der Biegeebene (A) mit einer der ein elastisches Element (4) aufweisenden Ebenen identifiziert, steht, die Biegeebene (A) während des Gebrauchs wahlweise mit der das elastische Element (4) aufweisenden Ebene, das die den Operationssitus identifizierende Abbildungselektrode (3) hält, fluchtbar ist.

2. Katheter nach Anspruch 1, **dadurch gekennzeichnet, daß** die elastischen Elemente (4) in Form von Plattenoder Stangenelementen hergestellt sind, die eine gewisse Biege-(Verdreh-)Festigkeit gegenüber der diese aufweisenden Ebenen haben.

3. Katheter nach Anspruch 1 oder 2, **dadurch gekennzeichnet, daß** die elastischen Elemente (4) von dem Schaft (2) des Katheters im wesentlichen büschelartig oder fontänenartig abzweigen.

4. Katheter nach einem der Ansprüche 1 oder 2, **dadurch gekennzeichnet, daß** die elastischen Elemente (4) von dem Schaft (2) des Katheters (1) abzweigen und dann an ihren distalen Enden an einem gemeinsamen Verbindungspunkt (6) zu einer im wesentlichen käfig- oder korbartigen Anordnung zusammenlaufen.

5. Katheter nach einem der vorhergehenden Ansprüche, **dadurch gekennzeichnet, daß** die elektrischen Lokalisierungskontaktelemente (9) den radialen Innenflächen der elastischen Elemente (4) zugeordnet sind.

6. Katheter nach einem der vorhergehenden Ansprüche, **dadurch gekennzeichnet, daß** die elastischen Elemente (4) entlang des Umfangs des Katheters (1) gleichwinklig voneinander beabstandet sind.

7. Katheter nach einem der vorhergehenden Ansprüche, **dadurch gekennzeichnet, daß** die entsprechenden elektrischen Lokalisierungskontaktelemente (9) und das weitere elektrische Kontaktelement (10) nahe der distalen Öffnung des Schaftes (2) des Katheters angeordnet sind.

8. Katheter nach einem der vorhergehenden Ansprüche, **dadurch gekennzeichnet, daß** das Halteelement (8) der Ablationselektrode (7) ein weiteres entsprechendes einzelnes Kontaktelement (10) hält, das sich in die Biegeebene (A) erstreckt.

9. Ein Katheter nach Anspruch 8, **dadurch gekennzeichnet, daß** das weitere elektrische Kontaktelement (10) an der Seite des Halteelementes (8), an der die Biegebewegung stattfindet, angeordnet ist.

10. Katheter nach einem der vorhergehenden Ansprüche, **dadurch gekennzeichnet, daß** die Länge des Halteelementes (8) der Ablationselektrode (7) wahlweise eingestellt werden kann.

## Revendications

1. Un cathéter pour le traitement de l'arythmie cardiaque, comprenant:
- une tige de cathéter (2) dont l'extrémité distale peut être amenée dans une chambre du coeur pour permettre son fonctionnement (V),
- une pluralité d'électrodes de mappage (3) portées par des éléments élastiques (4) qui se ramifient à partir de l'extrémité distale de la tige (2) et s'étendent dans des plans respectifs, et
- une électrode d'ablation (7) montée sur un élément support respectif (8) qui peut être disposé dans une position sensiblement centrale par rapport aux éléments élastiques (4) et peut s'incurver dans au moins un plan de cintrage respectif (A) afin de déplacer l'électrode d'ablation (7) en direction d'un site d'opératoire identifié par l'une des électrodes de mappage (3),
- l'élément support (8) pouvant être orienté de manière générale par rapport à la tige (2) de façon que le plan de cintrage (A) puisse être aligné sélectivement avec les plans contenant les éléments élastiques (4),
**caractérisé en ce que**:
- les éléments élastiques (4) portent des contacts électriques de localisation angulaire associés respectifs (9), et
- l'élément support (8) de l'électrode d'ablation (7) porte au moins un contact électrique respectif (10) qui peut être amené sélectivement dans des positions dans lesquelles il est en contact électrique avec les contacts électriques de localisation (9) des éléments élastiques (4) à la suite de l'orientation de l'élément support (8), chaque position dans laquelle le contact électrique respectif additionnel (10), dont il est prévu au moins un, est en contact électrique avec un contact électrique respectif de localisation (9) identifiant l'alignement du plan de cintrage (A) avec l'un des plans contenant un élément élastique (4) l'arrangement permettant au plan de cintrage (A) d'être aligné selectivement en utilisation avec le plan contenant l'élément élastique (4) qui porte l'électrode de mappage (3) identifiant le site opératoire.

2. Un cathéter selon la revendication 1, **caractérisé en ce que** les éléments élastiques (4) sont fabriqués sous la forme d'éléments en plaque ou en barre qui offrent une certaine résistance à la flexion (torsion) hors des plans respectifs qui les contiennent.

3. Un cathéter selon la revendication 1 ou la revendication 2, **caractérisé en ce que** les éléments élastiques (4) se ramifient à partir de la tige (2) du cathéter (1) sous la forme d'une configuration en gerbe ou en fontaine.

4. Un cathéter selon la revendication 1 ou la revendication 2, **caractérisé en ce que** les éléments élastiques (4) se ramifient à partir de la tige (2) du cathéter (1) et convergent ensuite à leurs extrémités distales jusqu'à un point de raccordement commun (6) sous une configuration générale en forme de cage ou de panier.

5. Un cathéter selon l'une quelconque des revendications précédentes, **caractérisé en ce que** les contacts électriques de localisation (9) sont associés avec les faces radialement intérieures des éléments élastiques (4).

6. Un cathéter selon l'une quelconque des revendications précédentes, **caractérisé en ce que** les éléments élastiques (4) sont régulièrement angulairement espacés autour du cathéter (1).

7. Un cathéter selon l'une quelconque des revendications précédentes, **caractérisé en ce que** les contacts électriques de localisation respectifs (9) et le contact électrique additionnel (10) sont situés adjacents à l'ouverture distale de la tige (2) du cathéter.

8. Un cathéter selon l'une quelconque des revendications précédentes, **caractérisé en ce que** l'élément support (8) de l'électrode d'ablation (7) comporte un unique contact électrique additionnel respectif (10) qui s'étend dans le plan de cintrage (A).

9. Un cathéter selon la revendication 8, **caractérisé en ce que** le contact électrique additionnel respectif (10) est disposé sur le côté de l'élément support (8) sur lequel le mouvement de flexion se produit.

10. Un cathéter selon l'une quelconque des revendications précédentes, **caractérisé en ce que** la longueur de l'élément support (8) de l'électrode d'ablation (7) est sélectivement réglable.
